# EUROPEAN PATENT SPECIFICATION

(11) **EP 4 076 298 B1**
(45) Date of publication and mention of the grant of the patent: **04.02.2026**
(21) Application number: 20839393.4
(22) Date of filing: 17.12.2020
(51) Int. Cl.: A61F 5/058

(54) **VARIABLE STIFFNESS ORTHOTIC SHELL**
ORTHESEHÜLLE MIT VARIABLER STEIFHEIT
COQUE ORTHÉTIQUE À RIGIDITÉ VARIABLE

(30) Priority: 17.12.2019 IT 201900024301
(43) Date of publication of application: 26.10.2022
(73) Proprietor: Scuola Superiore di Studi Universitari e di Perfezionamento Sant'Anna, 56127 Pisa (IT); INAIL - Istituto Nazionale per l'Assicurazione contro gli Infortuni sul Lavoro, 00187 Roma (RM) (IT)
(72) Inventor: BALDONI, Andrea, 56025 Pontedera (PI) (IT); CIANCHETTI, Matteo, 06131 Perugia - San Marco (IT); CREA, Simona, 55100 Lucca (IT); VITIELLO, Nicola, 56025 Pontedera (PI) (IT)
(74) Representative: ABM Agenzia Brevetti & Marchi
(86) International application number: PCT/IB2020/062108
(87) International publication number: WO 2021/124198

(56) References cited:
- US-A1- 2005 137 513
- US-A1- 2012 310 126
- US-A1- 2017 360 590
- US-A1- 2019 274 866

## Description

### Field of the invention

The present invention relates to the field of wearable robotics.

In particular, the invention relates to an orthotic shell with variable stiffness for anchoring an exoskeleton to an anatomical segment of a user.

### Description of the prior art

As is known, one of the most critical aspects in the design of a wearable robot concerns the human-robot physical interface, that is the interface that allows you to connect the robot to the anatomical segments. The creation of interfaces that allow a correct anchoring to the person is decisive for the purposes of comfort, acceptability and safety of the robot itself, regardless of the application for which the robot was designed (be it related to rehabilitation, assistance, or other).

In particular, an interface of this type must comply with a number of requirements, listed below:
- be sufficiently rigid to be able to anchor a robot and to be able to adequately support the constraint reactions generated by it;
- adapt to the specific anthropometry of the patient's hand;
- allow to lock the position of some joints in predetermined positions prescribed by the therapist during the rehabilitation sessions.

For example, a hand exoskeleton for finger rehabilitation must allow the wrist to be locked in the correct and physiological position for grasping movements.

In the prior art, various solutions are known for locking the movement of the wrist and thumb which generally make use of rigid braces. These solutions allow to block the desired joint by means of fabrics and semi-rigid components (typically plastic or aluminium) of enveloping shapes and to adapt the shape and size by means of adjustable bands or straps. However, these solutions are inappropriate in cases of particular deformities, for example in the case of pathological swellings.

US2017360590 discloses a deformable apparatus comprising locking sheets. The apparatus has a first state, in which the apparatus is deformable, and a second state, in which the apparatus has the desired shape and is less deformable than the first state. The device also includes an envelope that defines a chamber inside which the locking sheets are positioned in an at least partially overlapping configuration. Further, at least a portion of each locking sheet includes solid regions and open regions, and the solid regions are movable with respect to one another.

However, even this solution is difficult to adapt to complex anatomical geometries due to the low local deformability of the locking sheets.

### Summary of the invention

It is therefore a feature of the present invention to provide an orthotic shell that allows the anchoring of an exoskeleton to an anatomical segment of a user, appropriately supporting the constraint reactions generated by it.

It is also a feature of the present invention to provide such an orthotic shell for adapt to the specific anthropometry of the hand of the patient.

It is still a feature of the present invention to provide such an orthotic shell that allows to lock the position of a joint in a desired position.

These and other objects are achieved by an orthotic shell according to claims from 1 to 9.

The same objects are also achieved by an orthotic device according to claim 10.

### Brief description of the drawings

Further characteristic and/or advantages of the present invention are brighter with the following description of an exemplary embodiment thereof, exemplifying but not limitative, with reference to the attached drawings in which:
- Fig. 1A shows a plan view of a laminar element having three connecting portions;
- Fig. 1B shows a plan view of a laminar element having four connecting portions;
- Figs. 2A and 2B show, in perspective, the possible deformations of the laminar element of Fig. 1B;
- Fig. 3 shows, in perspective, an exemplary embodiment of the laminar structure of the orthotic shell, according to the present invention, comprising laminar elements having three connecting portions;
- Fig. 3A shows a detail of Fig. 3;
- Fig. 4 shows top plan view of an exemplary embodiment of the laminar structure of Fig. 3;
- Fig. 5 shows a plan view of an exemplary embodiment of the laminar structure comprising laminar elements with four connecting portions;
- Fig. 6 shows, in perspective, an exemplary embodiment of the laminar structure of Fig. 5 wherein ribbons are provided as fastening elements;
- Fig. 7 shows, in perspective, an exemplary embodiment of the laminar structure of Fig. 5 wherein nails are provided as fastening elements;
- Fig. 8 shows an exemplary embodiment of the orthotic shell comprising the laminar structure of Fig. 5 and the hermetic wrapper;
- Fig. 9 shows, in perspective, an exemplary embodiment of the orthotic shell comprising the laminar structure of Fig. 5 and a granular chamber;
- Fig. 10 shows, in perspective, an exemplary embodiment of the orthotic shell comprising two laminar structures and a plurality of granular chambers arranged on two layers;
- Fig. 11 shows an exemplary embodiment of the orthotic shell comprising the laminar structure of Fig. 5, a plurality of granular chambers and the hermetic wrapper;
- Fig. 12 shows, in perspective, an orthotic device for locking the wrist making use of the orthotic shell according to the present invention;
- Fig. 13 shows, in perspective, an orthotic device for locking the elbow making use of the orthotic shell according to the present invention.

### Description of a preferred exemplary embodiment

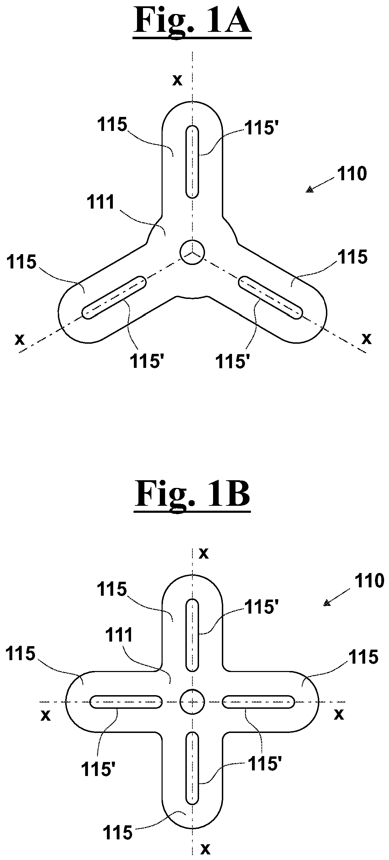

With reference to Figs. 1A to 7, the orthotic shell 10 according to the present invention comprises a laminar structure 100 comprising at least three overlapped layers consisting of respective laminar elements 110,120,130 of flexible material.

Hereinafter will be described the features of the laminar elements 110, valid *mutatis mutandis* for laminar elements 120 and 130.

In particular, in Figs. 1A and 1B two exemplary embodiments of the laminar element 110 are shown comprising a central portion 111 from which extend, respectively, 3 and 4 connecting portions 115 with elongated shape and having longitudinal directions x. Similarly, the laminar elements 120 and 130 comprise central portions 121,131 (not shown in the figures for simplicity) and connecting portions 125 and 135.

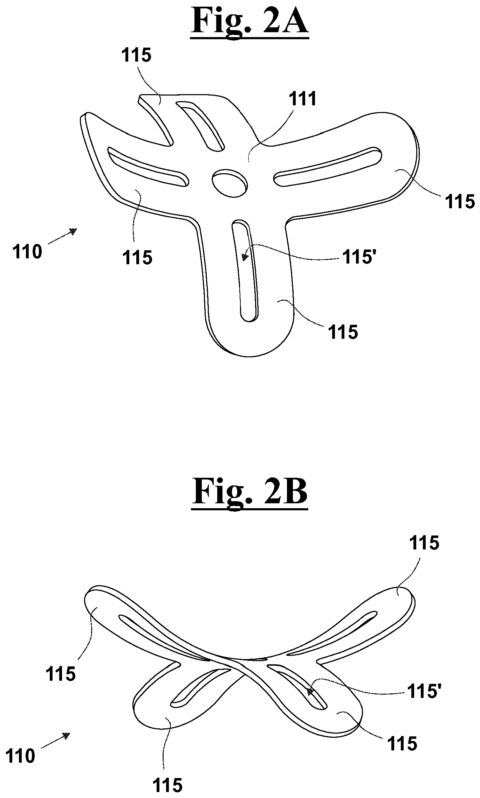

With reference to Figs. 2A and 2b, the laminar elements 110 are made of flexible material and the connecting portions 115 are configured for bending for making the laminar elements 110 pass between a planar geometry and a deformed geometry.

With particular reference to Fig. 2B, an exemplary embodiment of the laminar element 110 with 4 connecting portions 115 allows a "saddle" type deformation, which allows the laminar element 110 to be modelled on any three-dimensional surface.

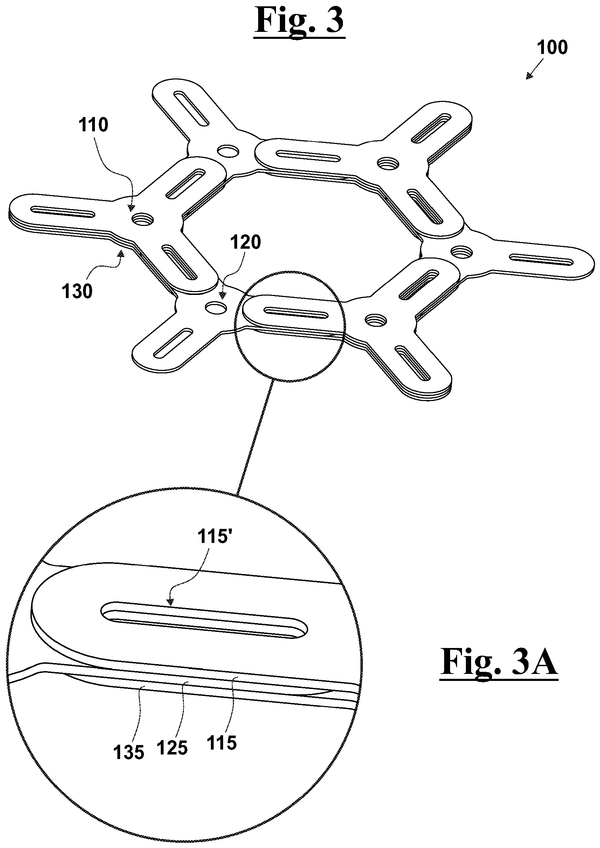

In the figures 3 and 3A a first exemplary embodiment is shown of the laminar structure 100, in which the laminar elements 110,120,130 of the type shown in Fig. 1A, i.e. comprising 3 connecting portions 115,125,135, are arranged on three parallel layers.

In particular the connecting portions 115,125,135 are to each other overlapped at respective elongated holes 115',125',135' (not all shown, for simplicity), in order to allow a connection of the laminar elements 110,120,130 of adjacent layers.

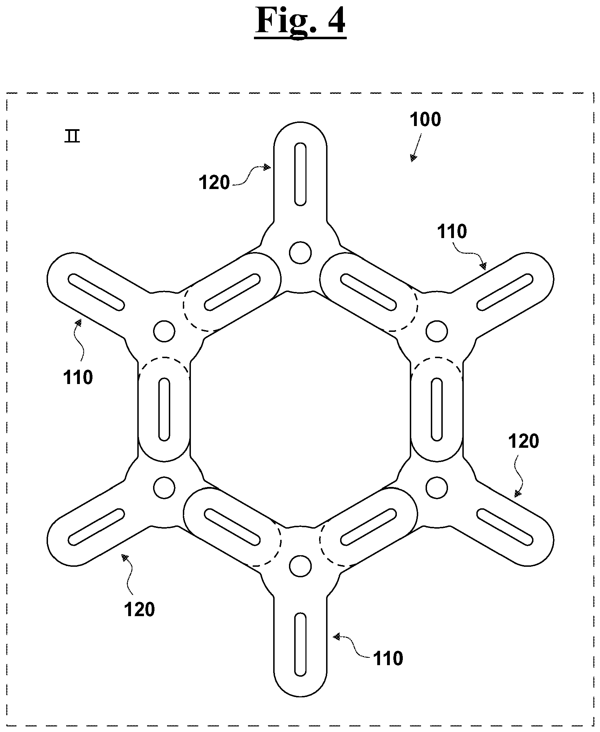

With reference to Fig. 4, when the laminar elements 110,120,130 are in the planar geometry, the laminar structure 100 has a two-dimensional shape and is substantially parallel to a reference plane ***π***. The bending of the connecting portions 115,125,135, on the other hand, allows the laminar structure 100 to have a three-dimensional shape capable of adapting to complex geometries.

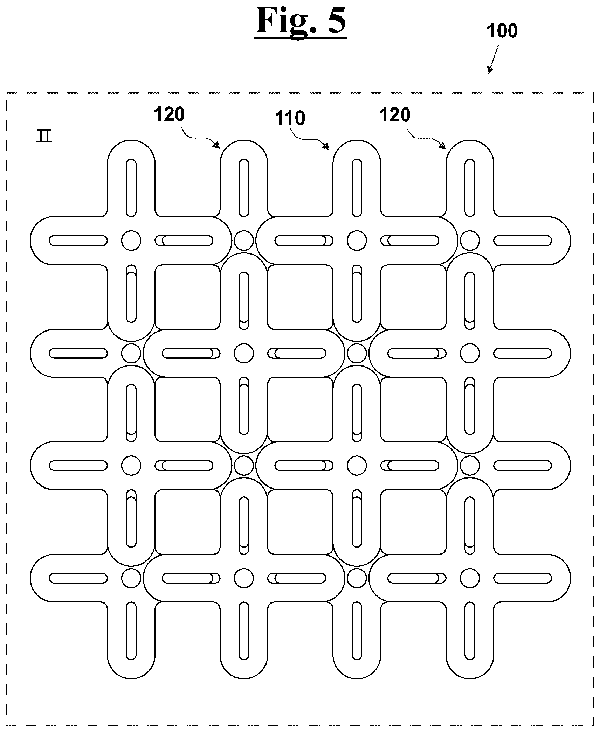

In the Figs. 5, 6 and 7 a second embodiment is shown of the laminar structure 100, in which the laminar elements 110,120,130 of the type shown in Fig. 1B, i.e. comprising 4 connecting portions 115,125,135, are arranged on five parallel layers. Such embodiment ensures to the laminar elements 110,120,130 to have complex shapes, such as the "saddle" one.

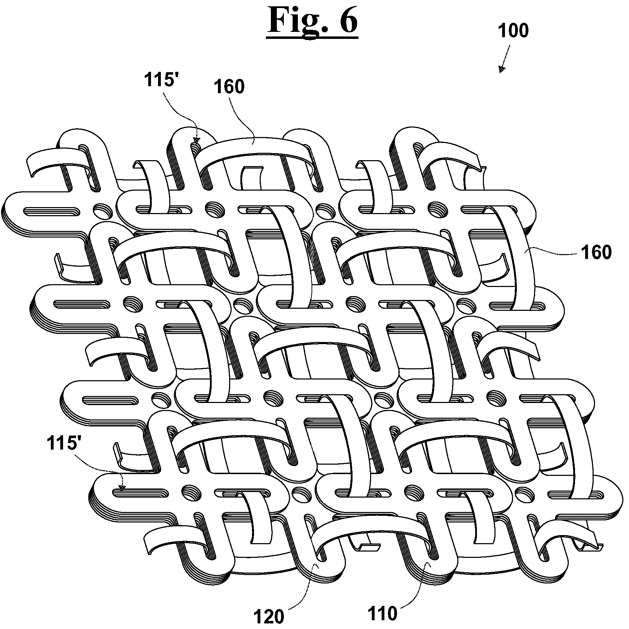

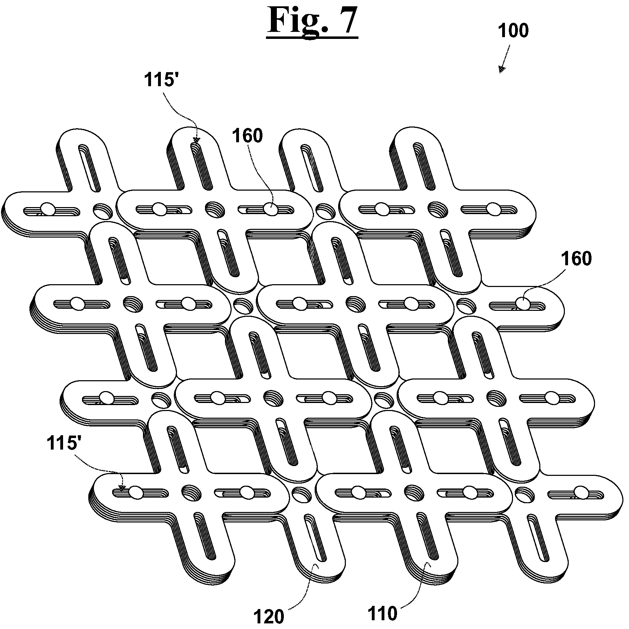

In particular, Figs. 6 and 7 show the fastening elements 160, respectively ribbons or nails, arranged to pass through the elongated holes 115' and the elongated holes below for constraining the laminar elements 110 with the underlying laminar elements 120,130.

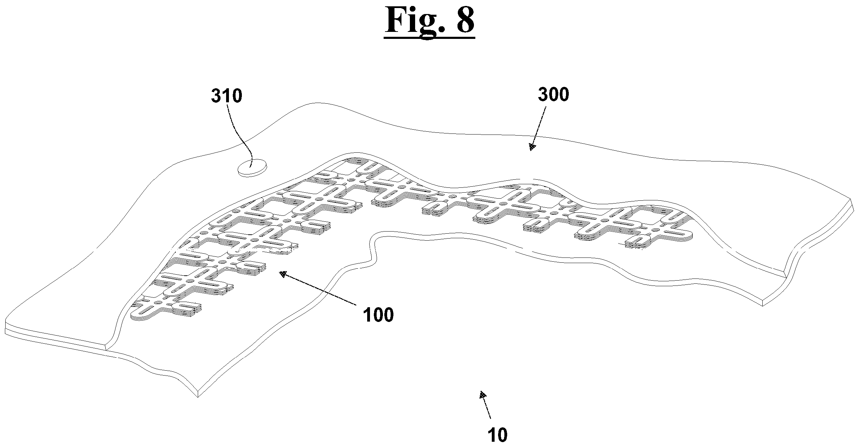

In Fig. 8 a first exemplary embodiment is shown of the orthotic shell 10 comprising a hermetic wrapper 300 arranged to contain the laminar structure 100 and to be put under vacuum by a pneumatic valve 310.

Such pneumatic valve 310 allows the air to be sucked in from the hermetic wrapper 300, making the orthotic shell 10 pass from a deformable configuration, where the connecting portions 115,125,135 can bend freely to make the laminar elements 110,120,130 pass between the planar geometry and the deformed geometry, to a rigid configuration, where the hermetic wrapper 300 is vacuum-packed and the connecting portions 115,125,135 are rigidly locked in their position.

In this way, the orthotic shell 10 can be shaped until the desired shape is obtained and subsequently be stiffened in this shape, thanks to the pressure that the vacuum produces between the laminar elements 110,120,130, preventing them from sliding together by friction. In this way it is possible to stiffen the orthotic shell 10 in even complex shapes, which adapt to anatomical portions such as the joints, to keep them locked in the desired position. This method is known in the literature as "layer jamming" or "laminar jamming". However, with respect to the prior art, the present invention, thanks to the particular conformation of the laminar elements 110, can adapt to complex three-dimensional geometries, such as those of a wrist or elbow joint.

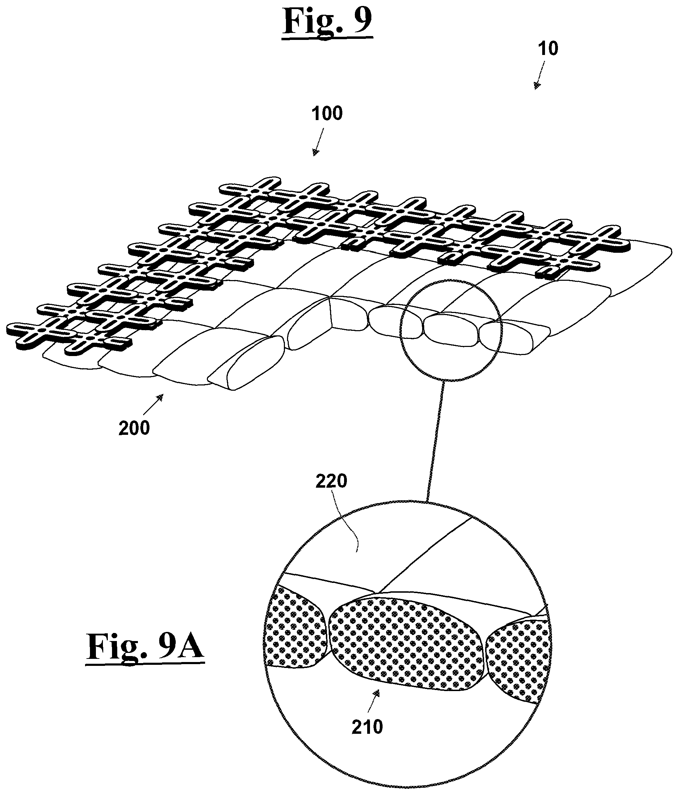

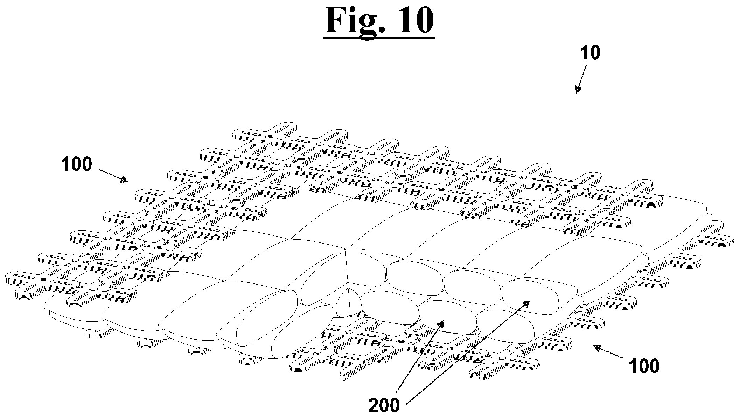

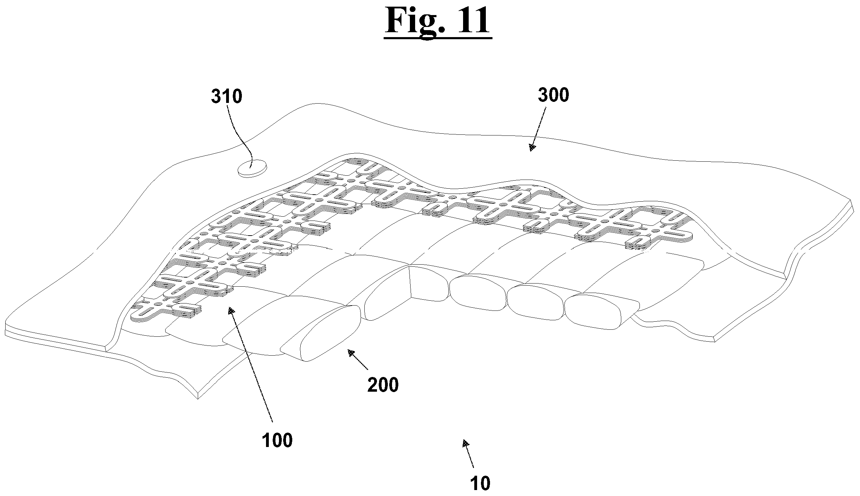

In Figs. 9 to 11 an embodiment is shown where the orthotic shell 10 also comprises one or two granular chambers 200 which constitute a "granular jamming" layer interposed between the two "layer jamming" layers. Each granular chamber 200 comprises a plurality of granules 210 of a predetermined size enclosed in a porous wrapper 220 adapted to allow the transpiration of a fluid and to prevent the escape of the granules 210 themselves.

In this way, when the orthotic shell 10 is placed under vacuum in the desired geometry, the granular chamber 200 can expel the air contained internally without causing the granules 210 to disperse, thanks to the porous envelope 220. The granular chamber 200 can then be stiffened together to the laminar structure 100, further increasing the solidity and non-deformability of the orthotic shell 10.

In the rigid configuration, therefore, the union of the granular chamber 200 and the laminar structure 100 allows the creation of a theoretical beam having the desired shape, allowing the orthotic shell 10 to correctly support both deflection and traction stresses.

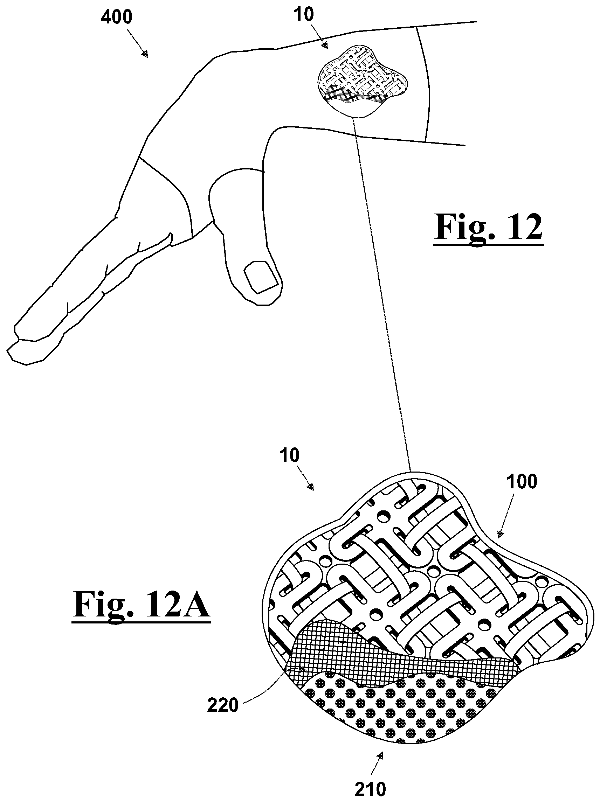

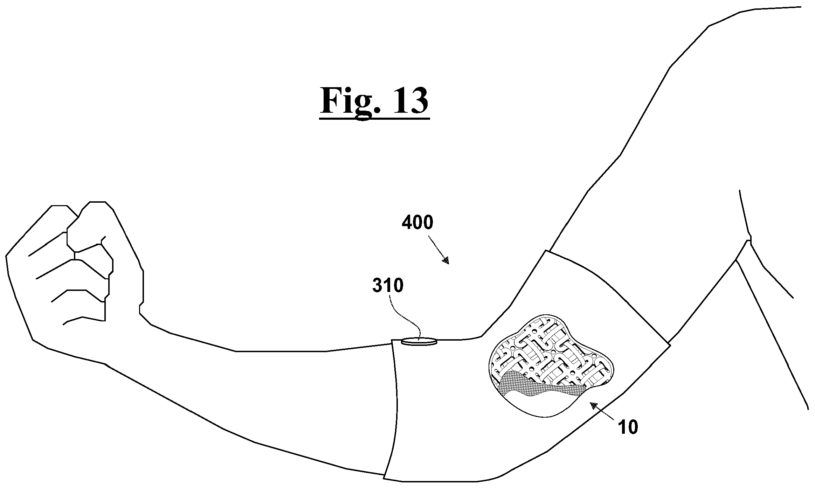

In Figs. 12 and 12A and in Figs. 13 and 13A, two possible applications of the orthotic shell 10 are shown inside orthotic devices 400 for locking, respectively, the wrist and elbow of a patient.

The foregoing description some exemplary specific embodiments will so fully reveal the invention according to the conceptual point of view, so that others, by applying current knowledge, will be able to modify and/or adapt in various applications the specific exemplary embodiments without further research and without parting from the invention, which is as defined by the appended claims. The means and the materials to realise the different functions described herein could have a different nature without, for this reason, departing from the field of the invention. it is to be understood that the phraseology or terminology that is employed herein is for the purpose of description and not of limitation.

## Claims

1. An orthotic shell (10) comprising:
- at least one laminar structure (100), comprising at least two overlapped layers, each layer comprising a plurality of laminar elements (110,120,130), said laminar elements (110,120,130) being made of flexible material and arranged to pass between a planar geometry, wherein said laminar structure (100) has a two-dimensional shape and is substantially parallel to a reference plane ***π***, and a deformed geometry, wherein said laminar structure (100) has a three-dimensional shape;
- a hermetic wrapper (300) arranged to contain said at least two layers, said hermetic wrapper (300) arranged to be put under vacuum;
wherein each laminar element (110,120,130) comprises a central portion (111,121,131) and ***n* ≥ 3** connecting portions (115,125,135), each connection portion (115,125,135) having an elongated shape that extends from said central portion (111,121,131) along a longitudinal direction x, wherein laminar elements (110,120,130) of a same layer are separated from each other,
wherein each connection portion (115) of each laminar element (110) of a first layer is arranged to overlap, at least partially, a connection portion (125) of a laminar element (120) of a second layer adjacent to said first layer in order to allow a connection of laminar elements (110,120,130) of adjacent layers,
and wherein said orthotic shell (10) is arranged to pass between a deformable configuration, wherein said connecting portions (115,125,135) are configured to bend for making said laminar elements (110,120,130) pass between said planar geometry and said deformed geometry, and a rigid configuration, wherein said hermetic wrapper (300) is vacuum-packed and said connecting portions (115,125,135) are subjected to mutual friction, rigidly locking each other in their position.

2. The orthotic shell (10), according to claim 1, wherein at least three layers are provided and wherein the laminar elements (110) of a first layer, in said planar geometry, have projections on said reference plane ***π*** coincident with the projections of laminar elements (130) of a third layer, not adjacent to said first layer.

3. The orthotic shell (10), according to claim 1, wherein each laminar element (110,120,130) comprises four connecting portions (115,125,135).

4. The orthotic shell (10), according to claim 1, wherein said connecting portions (115,125,135) comprise longitudinal axes x incident at said central portion (111,121,131) and wherein said connecting portions (115,125,135) are arranged in such a way that, in said planar geometry, said longitudinal axes x have, on said reference plane ***π*,** a relative angle equal to ***ϑ* = 360°/*n*.**

5. The orthotic shell (10), according to claim 1, wherein each connection portion (115,125,135) comprises an elongated hole (115',125',135') and wherein fastening elements (160) are also provided arranged to pass through said elongated holes (115',125',135') for constraining to each other said laminar elements (110, 120, 130) .

6. The orthotic shell (10), according to claim 5, wherein said fastening elements (160) are ribbons.

7. The orthotic shell (10), according to claim 1, wherein at least one granular chamber (200) is provided located in said hermetic wrapper (300), said granular chamber (200) comprising:
- a plurality of granules (210) of a predetermined size;
- a porous wrapper (220) arranged to contain said plurality of granules (210) and arranged to allow the passage of a fluid and to prevent the escape of said granules (210).

8. The orthotic shell (10), according to claim 7, wherein two laminar structures (100) and at least one granular chamber (200) located between said two laminar structures (100) are provided.

9. An orthotic device (400) for locking an anatomical portion of a patient comprising an orthotic shell (10) according to any of claims from 1 to 8.

## Patentansprüche

1. Orthesehülle (10), umfassend:
- mindestens eine laminare Struktur (100), die mindestens zwei überlappende Schichten umfasst, wobei jede Schicht eine Vielzahl von laminaren Elementen (110,120,130) umfasst, wobei die laminaren Elemente (110,120,130) aus flexiblem Material hergestellt sind und dazu angeordnet sind, zwischen einer planaren Geometrie, wobei die laminare Struktur (100) eine zweidimensionale Form aufweist und im Wesentlichen parallel zu einer Referenzebene *π* ist, und einer verformten Geometrie, wobei die laminare Struktur (100) eine dreidimensionale Form aufweist, zu verlaufen;
- eine hermetische Umhüllung (300), die dazu angeordnet ist, die mindestens zwei Schichten zu enthalten, wobei die hermetische Umhüllung (300) dazu angeordnet ist, unter Vakuum gesetzt zu werden;
wobei jedes laminare Element (110,120,130) einen zentralen Abschnitt (111,121,131) und *n*≥3 Verbindungsabschnitte (115,125,135) umfasst, wobei jeder Verbindungsabschnitt (115,125,135) eine längliche Form aufweist, die sich von dem zentralen Abschnitt (111,121,131) entlang einer Längsrichtung x erstreckt,
wobei laminare Elemente (110,120,130) einer gleichen Schicht voneinander getrennt sind,
wobei jeder Verbindungsabschnitt (115) jedes laminaren Elements (110) einer ersten Schicht dazu angeordnet ist, mindestens teilweise einen Verbindungsabschnitt (125) eines laminaren Elements (120) einer zweiten Schicht, die an die erste Schicht angrenzt, zu überlappen, um eine Verbindung von laminaren Elementen (110,120,130) angrenzender Schichten zu ermöglichen,
und wobei die Orthesehülle (10) dazu angeordnet ist, zwischen einer verformbaren Konfiguration, wobei die Verbindungsabschnitte (115,125,135) dazu konfiguriert sind, sich zu biegen, damit die laminaren Elemente (110,120,130) zwischen der planaren Geometrie und der verformten Geometrie verlaufen, und einer starren Konfiguration, wobei die hermetische Umhüllung (300) vakuumverpackt ist und die Verbindungsabschnitte (115,125,135) wechselseitiger Reibung ausgesetzt sind, wobei sie sich in ihrer Position starr verriegeln, zu verlaufen.

2. Orthesehülle (10) nach Anspruch 1, wobei mindestens drei Schichten bereitgestellt sind und wobei die laminaren Elemente (110) einer ersten Schicht in der planaren Geometrie Vorsprünge auf der Referenzebene *π* aufweisen, die mit den Vorsprüngen von laminaren Elementen (130) einer dritten Schicht zusammenfallen, die nicht an die erste Schicht angrenzen.

3. Orthesehülle (10) nach Anspruch 1, wobei jedes laminare Element (110,120,130) vier Verbindungsabschnitte (115,125,135) umfasst.

4. Orthesehülle (10) nach Anspruch 1, wobei die Verbindungsabschnitte (115,125,135) Längsachsen x umfassen, die auf den zentralen Abschnitt (111,121,131) treffen, und wobei die Verbindungsabschnitte (115,125,135) derart angeordnet sind, dass die Längsachsen x in der planaren Geometrie auf der Referenzebene *π* einen relativen Winkel gleich *ϑ* = 360°/*n* aufweisen.

5. Orthesehülle (10) nach Anspruch 1, wobei jeder Verbindungsabschnitt (115,125,135) ein längliches Loch (115',125',135') umfasst und wobei Befestigungselemente (160) ebenfalls bereitgestellt sind, die dazu angeordnet sind, durch die länglichen Löcher (115',125',135') zu verlaufen, um die laminaren Elemente (110,120,130) aneinander zu zwingen.

6. Orthesehülle (10) nach Anspruch 5, wobei die Befestigungselemente (160) Bänder sind.

7. Orthesehülle (10) nach Anspruch 1, wobei mindestens eine granulare Kammer (200) bereitgestellt ist, die sich in der hermetischen Hülle (300) befindet, wobei die granulare Kammer (200) Folgendes umfasst:
- eine Vielzahl von Granulaten (210) einer vorbestimmten Größe;
- eine poröse Umhüllung (220), die dazu angeordnet ist, die Vielzahl von Granulaten (210) zu enthalten, und dazu angeordnet ist, den Durchtritt eines Fluids zu ermöglichen und das Entweichen der Granulate (210) zu verhindern.

8. Orthesehülle (10) nach Anspruch 7, wobei zwei laminare Strukturen (100) und mindestens eine granulare Kammer (200), die sich zwischen den zwei laminaren Strukturen (100) befindet, bereitgestellt sind.

9. Orthesenvorrichtung (400) zum Verriegeln eines anatomischen Teils eines Patienten, umfassend eine Orthesehülle (10) nach einem der Ansprüche von 1 bis 8.

## Revendications

1. Coque orthétique (10) comprenant :
- au moins une structure laminaire (100), comprenant au moins deux couches superposées, chaque couche comprenant une pluralité d'éléments laminaires (110, 120, 130), lesdits éléments laminaires (110, 120, 130) étant faits d'un matériau flexible et agencés pour passer entre une géométrie plane, dans laquelle ladite structure laminaire (100) a une forme bidimensionnelle et est sensiblement parallèle à un plan de référence ***π*,** et une géométrie déformée, dans laquelle ladite structure laminaire (100) a une forme tridimensionnelle ;
- une enveloppe hermétique (300) agencée pour contenir lesdites au moins deux couches, ladite enveloppe hermétique (300) étant agencée pour être mise sous vide ;
dans laquelle chaque élément laminaire (110, 120, 130) comprend une partie centrale (111, 121, 131) et ***n* ≥ 3** parties de connexion (115, 125, 135), chaque partie de connexion (115, 125, 135) ayant une forme allongée qui s'étend à partir de ladite partie centrale (111, 121, 131) le long d'une direction longitudinale ***x*,**
dans laquelle les éléments laminaires (110, 120, 130) d'une même couche sont séparés les uns des autres,
dans laquelle chaque partie de connexion (115) de chaque élément laminaire (110) d'une première couche est agencée pour chevaucher, au moins partiellement, une partie de connexion (125) d'un élément laminaire (120) d'une deuxième couche adjacente à ladite première couche afin de permettre une connexion d'éléments laminaires (110, 120, 130) de couches adjacentes,
et dans laquelle ladite coque orthétique (10) est agencée pour passer entre une configuration déformable, dans laquelle lesdites parties de connexion (115, 125, 135) sont configurées pour se plier pour faire passer lesdits éléments laminaires (110, 120, 130) entre ladite géométrie plane et ladite géométrie déformée, et une configuration rigide, dans laquelle ladite enveloppe hermétique (300) est emballée sous vide et lesdites parties de connexion (115, 125, 135) sont soumises à un frottement mutuel, se verrouillant rigidement les unes les autres dans leur position.

2. Coque orthétique (10) selon la revendication 1, dans laquelle au moins trois couches sont prévues et dans laquelle les éléments laminaires (110) d'une première couche, dans ladite géométrie plane, ont des projections sur ledit plan de référence ***π*** coïncidant avec les projections des éléments laminaires (130) d'une troisième couche, non adjacente à ladite première couche.

3. Coque orthétique (10), selon la revendication 1, dans laquelle chaque élément laminaire (110, 120, 130) comprend quatre parties de connexion (115, 125, 135).

4. Coque orthétique (10), selon la revendication 1, dans laquelle lesdites parties de connexion (115, 125, 135) comprennent les axes longitudinaux ***x*** incidents au niveau de ladite partie centrale (111, 121, 131) et dans laquelle lesdites parties de connexion (115, 125, 135) sont agencées de telle manière que, dans ladite géométrie plane, lesdits axes longitudinaux x présentent, sur ledit plan de référence ***π*,** un angle relatif égal à *ϑ* = 360°/*n*.

5. Coque orthétique (10) selon la revendication 1, dans laquelle chaque partie de connexion (115, 125, 135) comprend un trou allongé (115', 125', 135') et dans laquelle des éléments de fixation (160) sont également prévus agencés pour passer à travers lesdits trous allongés (115', 125', 135') pour contraindre les uns aux autres lesdits éléments laminaires (110, 120, 130) .

6. Coque orthétique (10), selon la revendication 5, dans laquelle lesdits éléments de fixation (160) sont des rubans.

7. Coque orthétique (10), selon la revendication 1, dans laquelle au moins une chambre granulaire (200) est prévue située dans ladite enveloppe hermétique (300), ladite chambre granulaire (200) comprenant :
- une pluralité de granules (210) d'une taille prédéterminée ;
- une enveloppe poreuse (220) agencée pour contenir ladite pluralité de granules (210) et agencée pour permettre le passage d'un fluide et pour empêcher la fuite desdites granules (210).

8. Coque orthétique (10), selon la revendication 7, dans laquelle deux structures laminaires (100) et au moins une chambre granulaire (200) située entre lesdites deux structures laminaires (100) sont prévues.

9. Dispositif orthétique (400) pour verrouiller une partie anatomique d'un patient comprenant une coque orthétique (10) selon l'une quelconque des revendications 1 à 8.
